# EUROPÄISCHE PATENTSCHRIFT

(11) **EP 3 047 793 B1**
(45) Veröffentlichungstag und Bekanntmachung des Hinweises auf die Patenterteilung: **02.03.2022**
(21) Anmeldenummer: 16151696.8
(22) Anmeldetag: 18.01.2016
(51) Int. Cl.: A61B 5/00, A61F 13/02, G02B 6/00, A61F 13/00, A61M 27/00, F21V 8/00, G02B 6/02, A61F 13/84

(54) **WUNDVERBAND**
WOUND DRESSING
PANSEMENT

(30) Priorität: 20.01.2015 DE 102015100744
(43) Veröffentlichungstag der Anmeldung: 27.07.2016
(73) Patentinhaber: ATMOS MedizinTechnik GmbH & Co. KG, 79853 Lenzkirch (DE)
(72) Erfinder: Boysen, Björn, 79853 Lenzkirch (DE)
(74) Vertreter: Westphal, Mussgnug & Partner Patentanwälte mbB

(56) Entgegenhaltungen:
- EP-A1- 2 565 630
- WO-A1-2004/043542
- US-A- 5 183 323
- US-A- 5 432 876
- US-A1- 2007 239 232
- US-A1- 2009 177 051
- US-A1- 2013 035 629
- US-A1- 2013 325 087

## Beschreibung

Die Erfindung betrifft einen Wundverband mit den Merkmalen des Oberbegriffs des Patentanspruchs 1. Neben die traditionelle Funktion eines Wundverbands, die Wunde vor Umwelteinflüssen zu schützen, ist im Laufe der letzten Jahre immer stärker die Funktion getreten, eine kontrollierte Umgebung für die Wunde zu schaffen, eine Bestimmung des aktuellen Zustands einer Wunde zu ermöglichen und/oder die Beeinflussung der Bedingungen in der kontrollierten Umgebung in Abhängigkeit von diesem aktuellen Zustand durch ein Wundheilungstherapiegerät zu ermöglichen.

Ein Beispiel für solche Wundheilungstherapiegeräte sind Vakuumtherapiegeräte, wie sie beispielsweise aus der DE 2006/052745 A2 oder der DE 10 2012 201 390 A1 bekannt sind, mit denen die Atmosphäre im Bereich der Wunde kontrolliert wurde und eine Analyse des Zustands der Wunde durch eine Analyse der abgesaugten Wundflüssigkeit ermöglicht wurde.

Daneben ist es aus dem Stand der Technik aber auch bekannt, dass Beleuchtung, insbesondere mit Wärme- bzw. Infrarotstrahlung, Heilungsprozesse einer Wunde positiv beeinflussen kann. So offenbart beispielsweise die US 5 616 140 Bandagen, in welche lokal eine oder mehrere LEDs oder Laserdioden eingebettet sind, um eine solche Bestrahlungsmöglichkeit zu schaffen. In ähnlicher Weise ist es aus der WO 2004/043542 bekannt, lokale Leuchtelemente in einem Wundverband vorzusehen, zu welchen das Licht mittels einer Lichtleiterfaser geführt wird.

Aus der EP 2 565 630 A1 ist es bekannt, farbstoffdotierte, gelatinummantelte optische Fasern zur Insitu-Überwachung der Protease-Aktivität in Wunden zu verwenden, indem optische Eigenschaften der Ummantelung bestimmt werden. Ferner ist diesem Dokument zu entnehmen, dass solche Fasern in Wundverbände integriert werden können.

Aus der US 2009/0177051 A1 ist es bekannt, einen Wundverband mittels einer optischen Faser, deren Ende aufgefächert ist, zu beleuchten und Lichtempfänger vorzusehen, die unmittelbar am Wundverband angeordnet sein können oder die in weitere optische Fasern eingekoppeltes Licht auswerten.

Aus der US 2013/0035629 A1 ist eine optische Bandage zur Wundsterilisation bekannt, bei der eine Wundauflagefläche von hinten beleuchtet wird, was mittels eines mäanderförmig verlaufenden Lichtleiters erfolgen kann. Weiter hat man herausgefunden, dass durch Analyse des Brechungsindex einer Wunde Rückschlüsse auf den aktuellen Zustand der Wunde und den Verlauf des Heilungsprozesses gezogen werden können. Deshalb ist beispielsweise in der WO 2006/001759 A1 vorgeschlagen worden, durch eine Analyse der Variation der Lichtmenge, die durch eine Lichtleiterfaser hindurchtritt, in Abhängigkeit vom Brechungsindex der Umgebung der Lichtleiterfaser Rückschlüsse auf den Zustand der Wunde, der diesen Brechungsindex beeinflusst zu ziehen.

Die Aufgabe der Erfindung besteht darin, einen Wundverband bereitzustellen, bei dem einerseits eine nicht nur lokale Bestrahlungsmöglichkeit für die Wunde zur Förderung der Wundheilung gegeben ist und bei der die Notwendigkeit vermieden wird, zusätzlich zu den Mitteln, mit denen die Bestrahlung vorgenommen wird, eine separate Sensorik zur Analyse des Zustands der Wunde vorsehen zu müssen und der es zudem ermöglicht, mit seiner integrierten Sensorik Daten für die Ansteuerung eines Vakuumtherapiegerätes bereitzustellen.

Diese Aufgabe wird gelöst durch einen Wundverband mit den Merkmalen des Anspruchs 1. Vorteilhafte Weiterbildungen der Erfindung sind Gegenstand der abhängigen Patentansprüche.

Der erfindungsgemäße Wundverband weist eine Wundauflagefläche, mindestens eine Beleuchtungsvorrichtung zum Beleuchten der Wunde und mindestens eine Sensoranordnung, die für die Auswertung des Brechungsindex der Wunde die Strahlungsintensität am Ende der lichtleitenden Faser, der sie zugeordnet ist, detektiert, auf.

Erfindungswesentlich ist dabei, dass die Wundauflagefläche zumindest teilweise aus lichtleitenden Fasern besteht, die durch die Wundauflagefläche hindurch führen und aus denen zumindest abschnittsweise Licht in Richtung der Wunde austreten kann, dass an zumindest einigen, bevorzugt jedoch an allen dieser abschnittsweise Licht in Richtung der Wunde austreten kann, dass an zumindest einigen, bevorzugt jedoch an allen dieser lichtleitenden Fasern an ihrem einen Ende eine der Beleuchtungsvorrichtungen und an ihrem anderen Ende eine der Sensoranordnungen angeordnet ist und dass die jeweilige Sensoranordnung die Strahlungsmenge am Ende der lichtleitenden Faser, der sie zugeordnet ist, detektiert.

Gemäß der Erfindung bilden also die lichtleitenden Fasern einen Bestandteil der Wundauflagefläche und sind somit integraler Bestandteil des die Wundauflagefläche bildenden Gewebes, der die Wundauflagefläche komplett in einer Richtung durchsetzt, wobei diese Richtung insbesondere eine Richtung, die in der Ebene der Wundauflagefläche oder parallel dazu liegt, ist. Gleichzeitig werden die lichtleitenden Fasern nicht mehr, wie im Stand der Technik, lediglich als "Versorgungsleitung" für eine lokale Lichtquelle oder ein lokales Sensorelement verwendet, sondern aus ihnen wird unmittelbar und an einer Mehrzahl von Stellen entlang ihres Verlaufs das Licht in Richtung auf die Wunde hin ausgekoppelt, was eine flächige Beleuchtung der Wunde erlaubt.

Da zudem am einen Ende solcher lichtleitenden Fasern eine der Beleuchtungsvorrichtungen und an ihrem anderen Ende eine der Sensoranordnungen angeordnet ist und die jeweilige Sensoranordnung die Strahlungsmenge am Ende der lichtleitenden Faser, der sie zugeordnet ist, detektiert, kann zudem unmittelbar eine Aussage über den Brechungsindex der Wunde in der Umgebung dieser Faser gemacht werden, denn dieser beeinflusst die Menge des aus der lichtleitenden Faser austretenden Lichts. Durch Auswertung der detektierten Strahlungsmenge für unterschiedliche Fasern kann somit unmittelbar und ohne Entfernung des Wundverbands von der Wunde eine Aussage über den räumlichen Heilungsverlauf der Wunde gemacht werden. Durch Auswertung der zeitlichen Änderung der detektierten Strahlungsmenge für eine gegebene Faser kann ohne Entfernung des Wundverbands von der Wunde eine Aussage über den zeitlichen Heilungsverlauf der Wunde gemacht werden. Man kann also mit der vorgesehenen Sensoranordnung bzw. aus deren elektronisch auslesbaren Messdaten unmittelbar Aussagen über den Zustand der Wunde ableiten, die dann insbesondere auch zur automatischen Steuerung eines Vakuumtherapiegeräts verwendet werden können.

Die Lichtquelle kann dabei eine beliebige Lichtquelle, z.B. eine LED sein. Insbesondere kann es sich um eine Primärlichtquelle handeln, die direkt Licht erzeugt, oder um eine Sekundärlichtquelle, die an einem anderen Ort mit einer Primärlichtquelle erzeugtes Licht weiterleitet. Dabei kann jeder lichtleitenden Faser eine eigene Lichtquelle zugeordnet sein, es kann aber auch eine gemeinsame Lichtquelle direkt (also als Primärlichtquelle im oben definierten Sinn) oder indirekt mittels einer zwischengeschalteten lichtleitenden Faser (also als Sekundärlichtquelle im oben definierten Sinn) eine Beamsplitteranordnung die Beleuchtung aller lichtleitenden Fasern mit Licht aus derselben Quelle beleuchten. Dies ist oft vorteilhaft, weil dadurch ein Einfluss durch unterschiedliche reale Betriebsparameter der individuellen Lichtquellen auf das Ergebnis der Messung reduziert oder sogar eliminiert werden kann.

Die Sensoranordnung kann als einzelne, jeweils individuellen lichtleitenden Fasern oder Gruppen von lichtleitenden Fasern zugeordneten Photodetektoren beliebiger Bauart, aber auch als ein oder mehrere ortsauflösende Photodetektoren, bei denen einzelne Kanäle den lichtleitenden Fasern zugeordnet werden, realisiert werden.

Der Vollständigkeit halber sei an dieser Stelle darauf hingewiesen, dass bei auch bei einem noch nicht angelegten Wundverband, der also noch nicht an eine Wunde angrenzt, die Richtung auf die Wunde hin wohldefiniert ist. Bei Wundverbänden gibt es nämlich eine speziell ausgelegte Seite, welche bei bestimmungsgemäßem Gebrauch auf der Wunde aufliegt. Synonym zu der Phrase "in Richtung auf die Wunde hin" könnte also die Phrase "in der Richtung, in der bei bestimmungsgemäßem Gebrauch des Wundverbands die Wunde liegt" verwendet werden.

Das direkte Auskoppeln von Licht aus einem Lichtleiter ist auf mehrere Arten realisierbar. Bekanntermaßen weisen Lichtleiter, die üblicherweise einen kreisförmigen Querschnitt haben, einen zentralen Kern auf, der mit einem Fasermantel umgeben ist, dessen Brechungsindex kleiner ist als der des Kerns. Die Lichtleitung erfolgt durch Totalreflektion von Lichtstrahlen an der Grenzfläche zwischen zentralem Kern und Fasermantel. Optional kann zudem noch eine Ummantelung, z.B. aus einem Kunststoff wie Polyethylen vorgesehen werden, ggf. auf eine Zugentlastung, z.B. aus Stahl oder Kevlar-Litzen.

Eine Art, die Auskopplung von Licht aus dem Lichtleiter herbeizuführen, besteht darin, die Faser zu biegen. Bei Biegeradien im Bereich unterhalb einiger Zentimeter führt die Biegung dazu, dass die Totalreflektionsbedingung im Bereich der Biegung für einige Lichtstrahlen nicht mehr erfüllt ist, so dass es zum Lichtaustritt kommt.

Eine zweite Art, die Auskopplung von Licht aus dem Lichtleiter herbeizuführen, besteht darin, im Fasermantel und gegebenenfalls diesen umgebenden Ummantelungen Öffnungen vorzusehen. An diesen Stellen kann dann keine Totalreflektion mehr erfolgen und ein Lichtaustritt ist möglich.

Eine dritte Art, die Auskopplung von Licht aus dem Lichtleiter herbeizuführen, besteht darin, lokal nicht nur den Fasermantel zu entfernen, sondern zusätzlich die Geometrie des Faserkerns lokal zu verändern. Durch diese Geometrieveränderung des Faserkerns bzw. lokale Variation derselben wird es möglich, die lokal austretende Lichtmenge gezielt zu variieren.

In einer bevorzugten Ausgestaltung der Erfindung sind zumindest einige der lichtleitenden Fasern so modifiziert, dass die Austrittswahrscheinlichkeit des Lichts aus der lichtleitenden Faser in Richtung der Wunde von der Seite, an der die Beleuchtungsvorrichtung angeordnet ist zu der Seite, an der die Sensoranordnung angeordnet ist hin ansteigt. Idealerweise wird die Austrittswahrscheinlichkeit dabei so modifiziert, dass an einer gegebenen Austrittsstelle durch die erhöhte Austrittswahrscheinlichkeit die im Vergleich zur vorangegangenen Austrittsstelle wegen des dort ausgekoppelten Lichts geringer gewordenen Primärintensität kompensiert wird, so dass im Ergebnis eine homogene Beleuchtung der Wunde erreicht wird. Diese Maßnahme ist zudem gerade dann von besonderer Bedeutung, wenn die Daten der Sensoranordnung zu einer Aussage über den Zustand der Wunde herangezogen werden wollen, weil sonst unterschiedliche Stellen der Wunde unterschiedlich stark zum gemessenen Intensitätsverlust beitragen.

Konkret lässt sich dies für die drei unterschiedlichen Arten, die Auskopplung herbeizuführen, unterschiedlich realisieren. Benutzt man eine Biegung der Faser, so sollten zumindest die lichtleitenden Fasern, an deren einen Ende eine der Beleuchtungsvorrichtungen und an deren anderen Ende eine der Sensoranordnungen angeordnet ist, in Richtung senkrecht zur Auflagefläche des Wundverbands wellenförmig verlaufen, wobei ihre Krümmung an den Maxima und Minima der Wellenform in der Richtung von der Beleuchtungsvorrichtung zur Sensoranordnung monoton wächst und besonders bevorzugt streng monoton wächst. Der Vorteil dieser Variante besteht darin, dass eine Nachbearbeitung der lichtleitenden Fasern unterbleiben kann.

Führt man die Auskopplung durch das Entfernen des Fasermantel herbei, so können die lichtleitenden Fasern weiterhin in der Ebene oder parallel zu der Ebene der Auflagefläche des Wundverbandes verlaufen und der Fasermantel muss in Richtung auf die Wunde hin eine Öffnung aufweisen, deren Ausdehnung in Richtung senkrecht zur Verlaufsrichtung der lichtleitenden Faser von der Beleuchtungsvorrichtung zur Sensoranordnung hin wächst oder der Fasermantel muss mehrere Öffnungen aufweisen, deren jeweilige Flächen in Richtung von der Beleuchtungsvorrichtung zur Sensoranordnung hin monoton wachsen und besonders bevorzugt streng monoton wachsen.

Besonders bevorzugt ist eine Lösung, die auf einer Strukturierung des Faserkerns basiert, wenngleich diese zunächst einen erhöhten Bearbeitungsaufwand mit sich bringt. Diese sieht vor, dass die lichtleitenden Fasern in der Ebene oder parallel zu der Ebene der Auflagefläche des Wundverbandes verlaufen und dass die lichtleitenden Fasern an der Seite, die von der Wunde weg zeigt, Ausnehmungen aufweist, die sich in den Kern der lichtleitenden Faser hinein erstrecken und jeweils eine in der Richtung von der Beleuchtungsvorrichtung zur Sensoranordnung hin abfallende Rampe aufweisen, wobei die Steilheit des Abfalls der jeweiligen abfallenden Rampe in der Richtung von der Beleuchtungsvorrichtung zur Sensoranordnung hin von Ausnehmung zu Ausnehmung monoton wächst und besonders bevorzugt streng

monoton wächst. Durch die Änderung der Rampensteilheit wird die gewünschte Änderung der Austrittswahrscheinlichkeit herbeigeführt, wobei eine wesentlich bessere Homogenität der abgestrahlten Lichtintensität über den gesamten Wundverband erreicht werden kann, als dies bei den lokalen Modifikationen des Fasermantels oder den notwendigerweise diskret liegenden Extrema einer gewellt geführten lichtleitenden Faser der Fall ist.

Besonderes bevorzugt ist es, wenn der Wundverband mindestens eine lichtleitende Referenzfaser ohne Abschnitt, in dem Licht in Richtung der Wunde austreten kann, aufweist, an deren einem Ende eine Beleuchtungsvorrichtung und an deren anderem Ende eine Sensoranordnung angeordnet ist. Wie durch den Begriff "Referenzfaser" angedeutet wird, kann eine solche lichtleitende Faser dazu dienen, Änderungen der von der Sensoranordnung detektierten Lichtintensität, die nicht vom Verlauf der Wundheilung verursacht wurden, sicher zu identifizieren. Solche Änderungen können beispielsweise durch unterschiedliche Anbringung bzw. Lage des Wundverbands hervorgerufen werden. Die-Messung der Intensitätsänderung in den Referenzfasern gibt Aufschluss über solche Intensitätsänderungen.

Bei der nicht beanspruchten Verwendung eines erfindungsgemäßen Wundverbands mit einem Wundheilungstherapiegerät, das insbesondere als Vakuumtherapiegerät ausgeführt sein kann, werden mit der Sensoranordnung des Wundverbands gewonnene Daten zur Steuerung des Wundheilungstherapiegeräts, z.B. des Vakuumtherapiegeräts, verwendet.

Die Erfindung wird nachfolgend anhand von Figuren, die Ausführungsbeispiele zeigen, näher erläutert. Es zeigt:
- Fig.1:: den schematischen Aufbau einer ersten Variante eines erfindungsgemäßen Wundverbands,
- Fig.2:: den schematischen Aufbau einer zweiten Variante eines erfindungsgemäßen Wundverbands,
- Fig.3:: den schematischen Aufbau einer dritten Variante eines erfindungsgemäßen Wundverbands,
- Fig.4:: Einen Ausschnitt aus einer Wundauflagefläche eines Wundverbands mit eingewebten lichtleitenden Fasern,
- Fig.5:: einen Lichtleiter mit einer lokalen Modifikation des Faserkerns,
- Fig.6:: einen Querschnitt durch einen Lichtleiter mit einer bevorzugten lokalen Modifikation des Faserkerns in seiner Erstreckungsrichtung
- Fig.7:: einen Lichtleiter mit einer ersten Variante einer lokalen Veränderung des Fasermantels, und
- Fig.8:: einen Lichtleiter mit einer zweiten Variante einer lokalen Veränderung des Fasermantels.

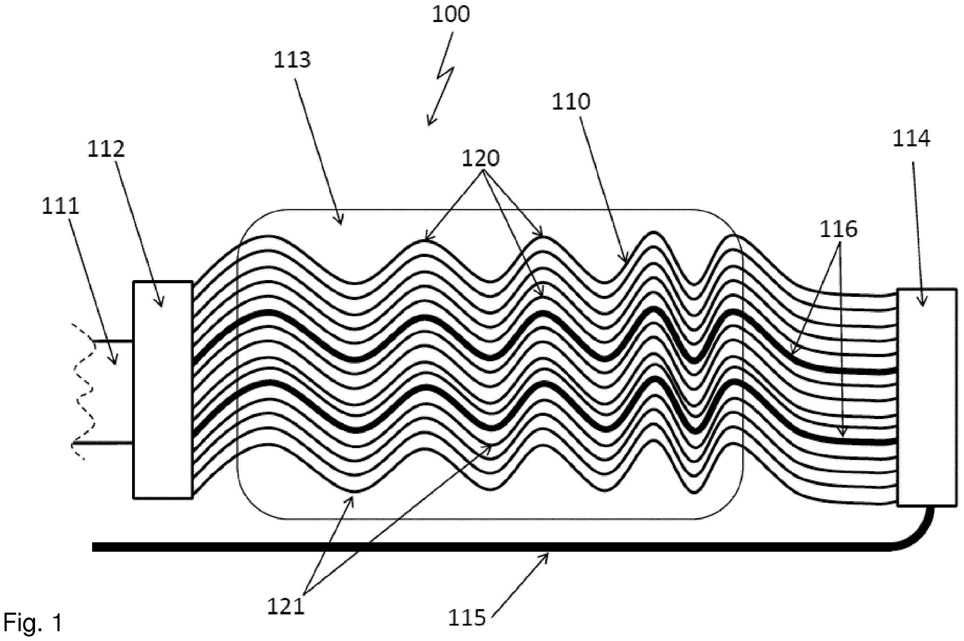

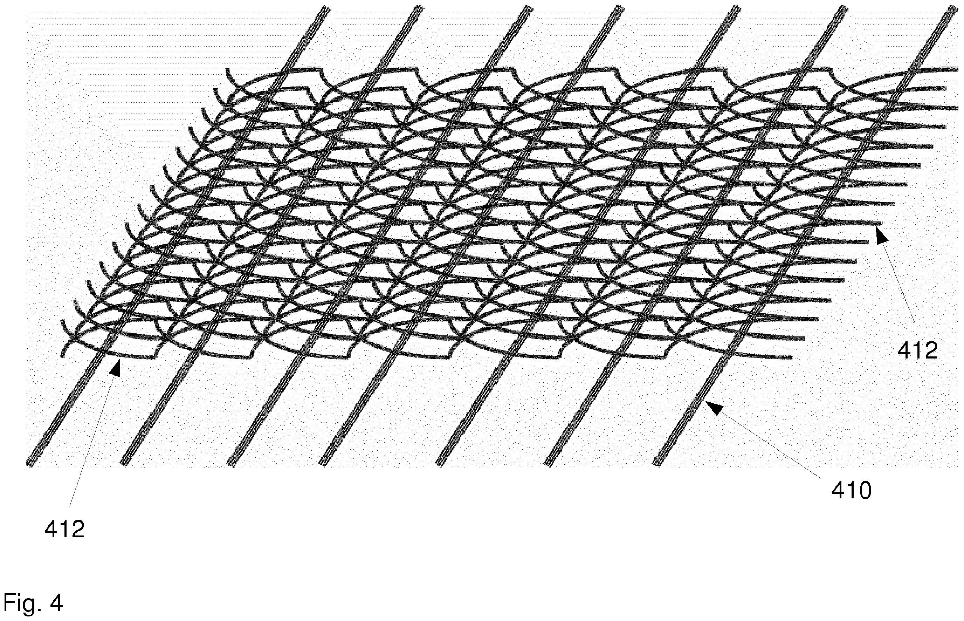

Figur 1 zeigt den schematischen Aufbau einer ersten Variante eines erfindungsgemäßen Wundverbands 100 mit Wundauflagefläche 113, die von mehreren parallel zueinander verlaufenden lichtleitenden Fasern 110 durchsetzt wird, welche, wie unten anhand der Figur 4 näher erläutert wird, einen Bestandteil der Wundauflagefläche 113 bilden, so dass die Wundauflagefläche 113 zumindest teilweise durch lichtleitende Fasern 110 gebildet wird. Schematisch an dieser Darstellung ist insbesondere, dass sie den strukturellen Aufbau der Wundauflagefläche 113 nicht richtig wiedergibt, sondern lediglich den Verlauf der lichtleitenden Fasern 110 in diesem Bereich und den Umriss der Wundauflagefläche zeigt. Der strukturelle Aufbau der Wundauflagefläche ist in Figur 4 anhand eines Ausschnitts einer solchen dargestellt. Man entnimmt der Figur 4, dass die lichtleitenden Fasern 410 ein integraler Bestandteil des die Wundauflagefläche 413 bildenden Gewebes ist und in dem dort gezeigten Beispiel dessen Kettfäden bilden, während die Schussfäden 412 aus einem anderen Material bestehen. Es können aber alternativ auch die Schussfäden durch lichtleitende Fasern gebildet werden, was insbesondere Vorteile hat, wenn ein Auskopplung des Lichtes durch Krümmung herbeigeführt werden soll, oder Kett- und Schussfäden können aus lichtleitenden Fasern bestehen.

Am einen Ende der lichtleitenden Fasern 110 werden diese mit Licht versorgt, das in diesem Beispiel von der Primärlichtquelle über ein als Sekundärlichtquelle dienendes Faserbündel 111 zugeführt und über eine Beamsplitteranordnung 112 in die einzelnen lichtleitenden Fasern 110 eingespeist wird. Am anderen Ende der lichtleitenden Fasern 110 ist ein Sensoranordnung 114 angeordnet, die die Lichtintensität am Ende der jeweiligen Faser misst, woraus insbesondere Rückschlüsse auf den Brechungsindex in der Umgebung der lichtleitenden Fasern 110 gezogen werden können. Die Sensoranordnung 114 ist über eine Rückkopplungsleitung 115 mit einer nicht dargestellten Steuer- und/oder Auswerteelektronik verbunden, welche die gemessenen Daten ausliest, aufbereitet und anzeigt und/oder in Steuersignale, beispielsweise ein Steuerkommando für ein Vakuumtherapiegerät umsetzt.

Wie man in Figur 1 erkennt, verlaufen die einzelnen lichtleitenden Fasern 110 annähernd parallel zueinander und wellenförmig in Richtung senkrecht zur Wundauflagefläche 113 des Wundverbands 100 und weisen somit Maxima 120 und Minima 121 auf, deren Krümmung von der Richtung des als Sekundärlichtquelle dienenden Faserbündels 111 hin zur Sensoranordnung monoton, insbesondere streng monoton wächst.

Ferner weist der in Figur 1 dargestellte Wundverband Referenzfasern 116 auf, welche dicker gezeichnet sind als die lichtleitenden Fasern 110, und aus denen kein Licht in Richtung der Wunde austreten kann. Dies kann insbesondere realisiert werden, wenn die Referenzfasern 116 eine Ummantelung aufweisen, die den Lichtaustritt in Richtung auf die Wunde hin verhindert. Durch die Messung der Lichtintensität am Ende der Referenzfasern 116 können Einflüsse auf die Intensitätsänderung, die nicht von der Entwicklung der Wundheilung abhängen, sicher identifiziert und bei der Auswertung des Heilungsprozesses berücksichtigt werden.

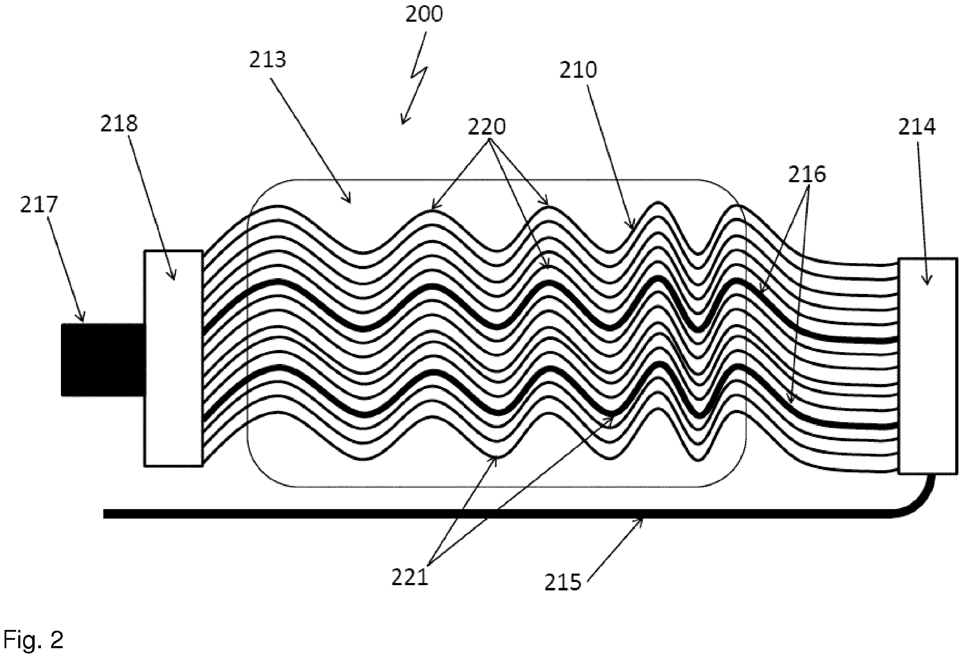

Die Ausführungsform eines Wundverbandes 200 gemäß Figur 2 ist weitgehend identisch zur Ausführungsform gemäß Figur 1 und ebenfalls aus den bei der Diskussion der Figur 1 genannten Gründen als schematisch anzusehen. Der Unterschied besteht darin, dass am Ende der lichtleitenden Fasern 210 bzw. Referenzfasern 216 keine Sekundärlichtquelle angeordnet ist, sondern die Lichtquelle 218 mit Stromversorgung 217. Der verbleibende Aufbau ist analog zum Aufbau gemäß Figur 1, wobei sich die verwendeten Bezugszeichen durch Addition von 100 aus den Bezugszeichen der Figur 1 ergeben.

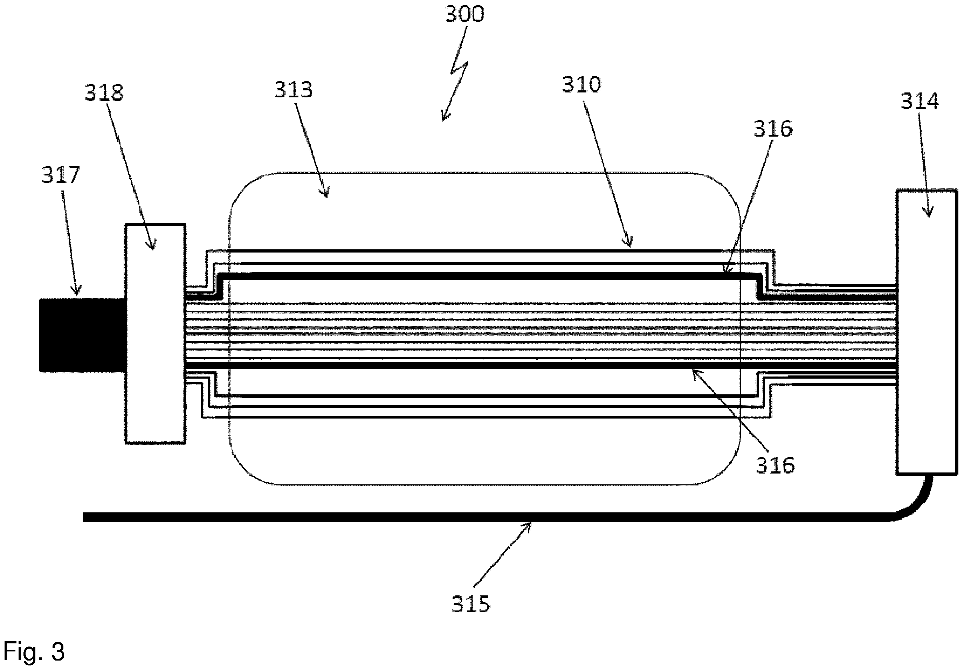

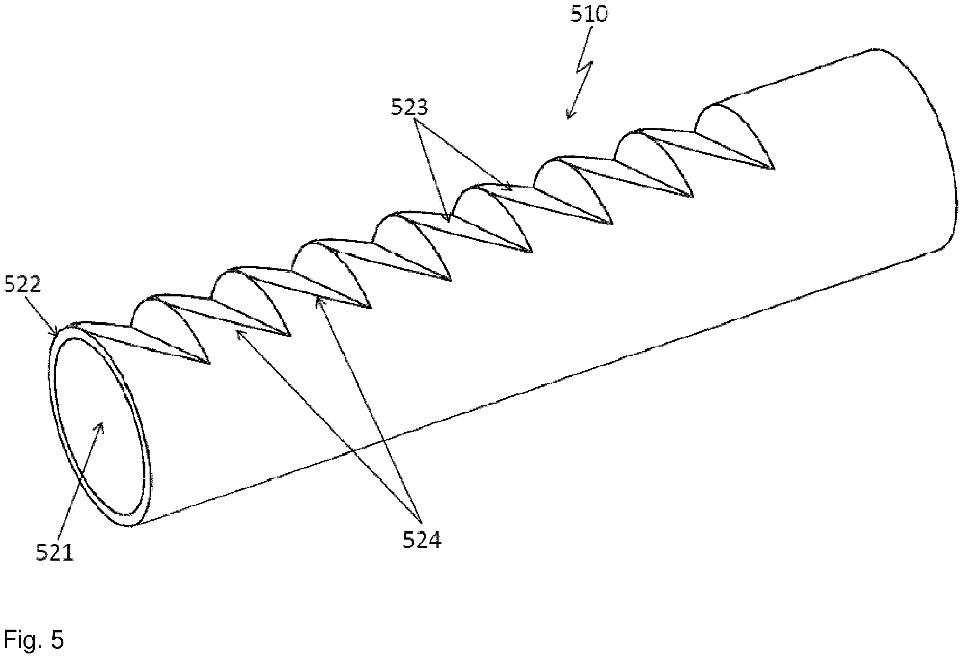

Die Ausführungsform eines Wundverbandes 300 gemäß Figur 3 ist weitgehend identisch zur Ausführungsform gemäß Figur 2 und ebenfalls aus den bei der Diskussion der Figur 1 genannten Gründen als schematisch anzusehen. Der Unterschied zum Wundverband 200 gemäß Figur 2 besteht darin, dass die lichtleitenden Fasern 310 und die Referenzfasern 316 nicht wellenförmig, sondern gerade verlaufen und somit der Lichtaustritt durch Modifikation des Fasermantels oder des Fasermantels und des Faserkerns bewirkt wird. Wie dies genau erfolgen kann, wird weiter unten anhand der Figuren 5 bis 8 näher erläutert. Der verbleibende Aufbau ist analog zum Aufbau gemäß Figur 2, wobei sich die verwendeten Bezugszeichen durch Addition von 100 aus den Bezugszeichen der Figur 2 ergeben. Natürlich kann auch die Ausführungsform gemäß Figur 3 statt mit einer Primärlichtquelle, wie in Figur 2 dargestellt, mit einer Sekundärlichtquelle in Form eines Faserbündels, wie in Figur 1 dargestellt, ausgeführt werden.

Die Figuren 5 bis 8 zeigen unterschiedliche Arten, die Auskopplung ohne Krümmung herbeizuführen. Die in Figur 5 dargestellte lichtleitende Faser 510 weist einen Kern 521 und einen Fasermantel 522 auf. Auf der der gewünschten Austrittsrichtung gegenüberliegenden Seite sind Ausnehmungen 523 vorgesehen, die sich durch den Fasermantel 522 hindurch in den Kern 521 der lichtleitenden Faser 510 hinein erstrecken und jeweils eine in die Richtung, in der die lichtleitende Faser 510 das Licht leitet, was bei der Verwendung einer solchen Faser in einem erfindungsgemäßen Wundverband der Richtung von der Beleuchtungsvorrichtung zur Sensoranordnung hin entspricht, hin abfallende Rampe 524 aufweisen. Die Wahl der Schräge der Rampe 524 beeinflusst den Anteil des Lichtes, der dann nach der Reflektion an der Rampe 524 auf der gegenüberliegenden Seite der lichtleitenden Faser 510 die Reflektionsbedingung nicht mehr erfüllt und ausgekoppelt wird.

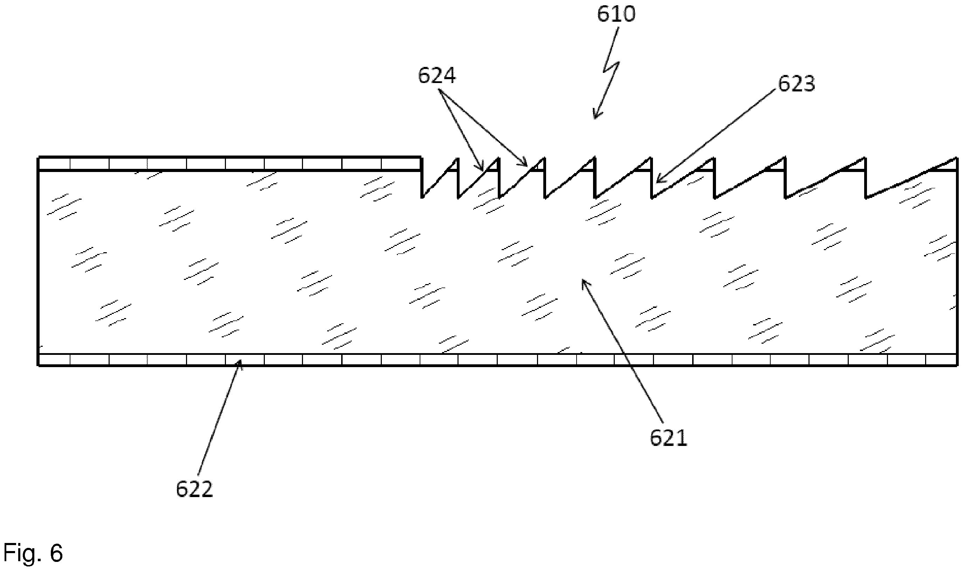

Die in Figur 6 im Querschnitt dargestellte lichtleitenden Faser 610 mit Kern 621 und Fasermantel 622 bildet die Ausführungsform der Figur 5 dadurch weiter, dass die Steilheit der Rampen 624 der Ausnehmungen 623 variiert, und zwar so, dass die Steilheit des Abfalls der jeweiligen abfallenden Rampe 624 in der Richtung, in der die lichtleitende Faser 610 das Licht leitet, was bei der Verwendung einer solchen Faser in einem erfindungsgemäßen Wundverband der Richtung von der Beleuchtungsvorrichtung zur Sensoranordnung hin entspricht, von Ausnehmung zu Ausnehmung monoton, insbesondere streng monoton wächst. Auf diese Weise kann die Intensität des abgestrahlten Lichtes über einen Streckenabschnitt in guter Näherung konstant gehalten werden.

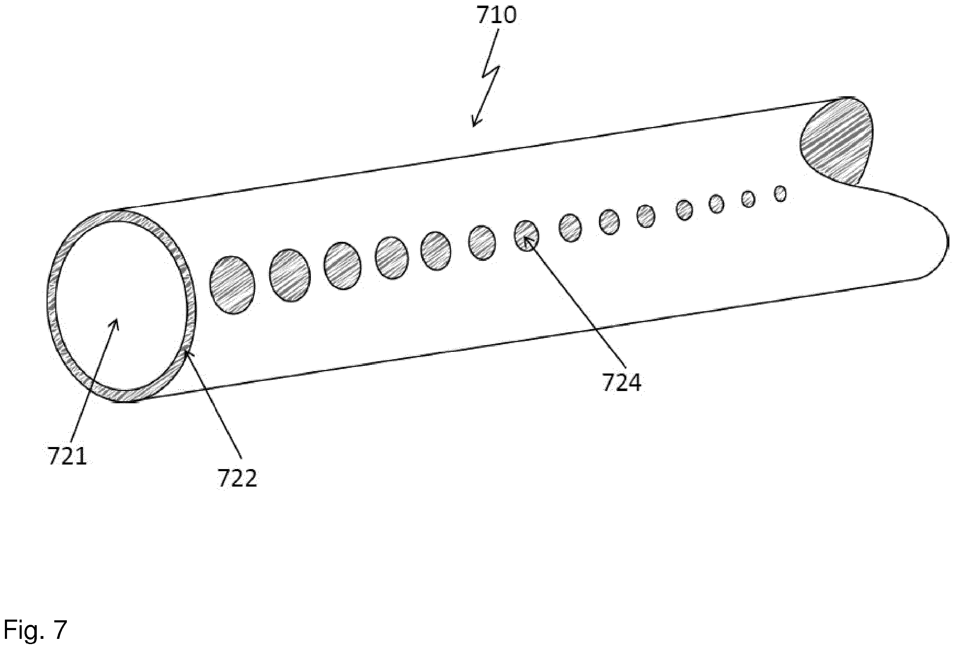

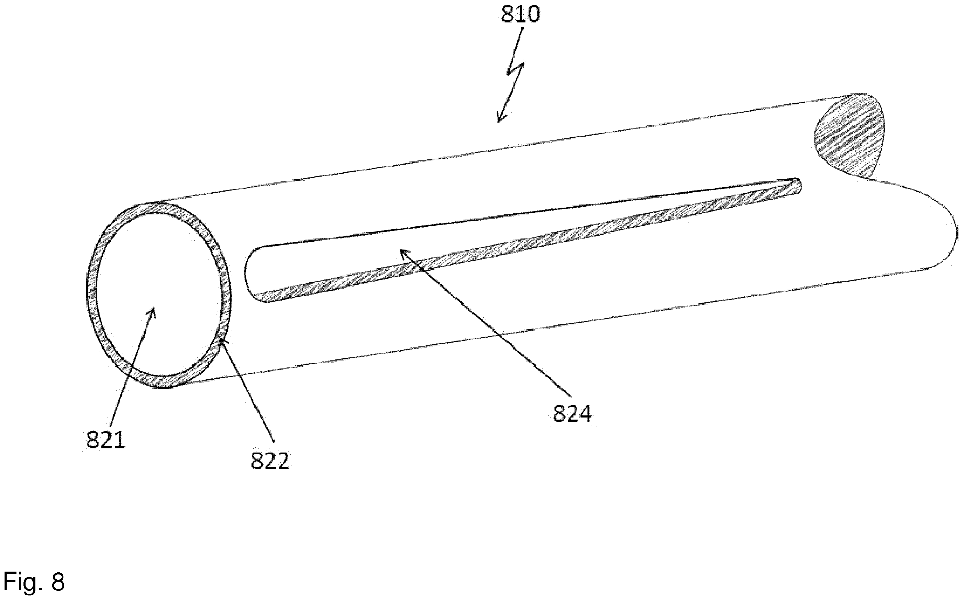

Eine solche näherungsweise Konstanz der abgestrahlten Lichtintensität lässt sich auch ohne Modifikation von Faserkernen erreichen, wie in den Figuren 7 und 8 dargestellt ist. Figur 7 zeigt eine lichtleitende Faser 710 mit Kern 721 und Fasermantel 722. Dabei weist der Fasermantel 722 in Richtung auf die Wunde hin mehrere Öffnungen 724 auf, in der die lichtleitende Faser 710 das Licht leitet, was bei der Verwendung einer solchen Faser in einem erfindungsgemäßen Wundverband der Richtung von der Beleuchtungsvorrichtung zur Sensoranordnung hin entspricht. Dementsprechend steigt die Auskoppelwahrscheinlichkeit an.

Figur 8 zeigt eine lichtleitende Faser 810 mit Kern 821 und Fasermantel 822. Dabei weist der Fasermantel 822 in Richtung auf die Wunde hin eine Öffnung 824 auf, deren Ausdehnung in Richtung senkrecht zur in der die lichtleitende Faser 810 das Licht leitet, was bei der Verwendung einer solchen Faser in einem erfindungsgemäßen Wundverband der Richtung von der Beleuchtungsvorrichtung zur Sensoranordnung hin entspricht, monoton, insbesondere streng monoton wächst. Dementsprechend steigt die Auskoppelwahrscheinlichkeit an.

### Bezugszeichenliste

- 100,200,300: Wundverband
- 110,210,310,410, 510,610,710,810: lichtleitende Faser
- 111: Faserbündel
- 112: Beamsplitteranordnung
- 113,213,313: Wundauflagefläche
- 114,214,314: Sensoranordnung
- 116,216,316: Referenzfaser
- 120,220: Maximum
- 121,221: Minimum
- 217,317: Stromversorgung
- 218,318: Lichtquelle
- 412: Schussfaden
- 521,621,721,821: Kern
- 522,622,722,822: Fasermantel
- 523,623: Ausnehmung
- 524,624: Rampe
- 724,824: Öffnung

## Patentansprüche

1. Wundverband (100,200,300) mit einer Wundauflagefläche (113,213,313), mit mindestens einer Beleuchtungsvorrichtung zum Beleuchten der Wunde und mit mindestens einer Sensoranordnung (114,214,314), wobei die Wundauflagefläche (113,213,313) zumindest teilweise durch lichtleitende Fasern (110,210,310) gebildet wird, die ein integraler Bestandteil des die Wundauflagefläche (113, 213, 313) bildenden Gewebes sind und seine Kett- und/oder Schussfäden bilden, die durch die Wundauflagefläche (113,213,313) hindurchführen, so dass sie die Wundauflagefläche (113, 213, 313) komplett in einer Richtung durchsetzen, die in der Ebene der Wundauflagefläche oder parallel dazu liegt und aus denen unmittelbar und an einer Mehrzahl von Stellen entlang ihres Verlaufs das Licht in Richtung auf die Wunde hin ausgekoppelt wird, so dasszumindest abschnittsweise Licht in Richtung der Wunde austreten kann, dass an zumindest einigen dieser lichtleitenden Fasern (110,210,310) an ihrem einen Ende eine Beleuchtungsvorrichtung und an ihrem anderen Ende eine Sensoranordnung (114,214,314) angeordnet ist und dass die jeweilige Sensoranordnung (114,214,314) für eine Auswertung des Berechnungsindex der Wunde die Strahlungsintensität am Ende der lichtleitenden Faser (110,210,310), der sie zugeordnet ist, detektiert.

2. Wundverband (100,200,300) nach Anspruch 1, wobei zumindest einige der lichtleitenden Fasern (110,210,310) aus denen zumindest abschnittsweise Licht in Richtung der Wunde austreten kann so modifiziert sind, dass die Austrittswahrscheinlichkeit des Lichts aus der lichtleitenden Faser (110,210,310) in Richtung der Wunde von der Seite, an der die Beleuchtungsvorrichtung angeordnet ist zu der Seite, an der die Sensoranordnung (114,214,314) angeordnet ist hin ansteigt.

3. Wundverband (100,200) nach Anspruch 2, wobei zumindest die lichtleitenden Fasern (110,210), an deren einen Ende eine Beleuchtungsvorrichtung und an deren anderen Ende eine Sensoranordnung (114,214) angeordnet ist und aus denen zumindest abschnittsweise Licht in Richtung der Wunde austreten kann, in Richtung senkrecht zur Wundauflagefläche (113,213) des Wundverbands (100,200) wellenförmig verlaufen, wobei ihre Krümmung an den Maxima (120,220) und Minima (120,220) der Wellenform in der Richtung von der Beleuchtungsvorrichtung zur Sensoranordnung (114,214) monoton wächst.

4. Wundverband (300) nach Anspruch 2, wobei die lichtleitenden Fasern (310,710,810) aus denen zumindest abschnittsweise Licht in Richtung der Wunde austreten kann in der Ebene oder parallel zu der Ebene der Wundauflagefläche (313) des Wundverbandes (300) verlaufen und dass der Fasermantel (722,822) entweder in Richtung auf die Wunde hin eine Öffnung (824) aufweist, deren Ausdehnung in Richtung senkrecht zur Verlaufsrichtung der lichtleitenden Faser (810) von der Beleuchtungsvorrichtung zur Sensoranordnung (313) hin wächst oder mehrere Öffnungen (724) aufweist, deren jeweilige Flächen in Richtung von der Beleuchtungsvorrichtung zur Sensoranordnung (314) hin monoton wachsen.

5. Wundverband (300) nach Anspruch 2, wobei die lichtleitenden Fasern (310,610) aus denen zumindest abschnittsweise Licht in Richtung der Wunde austreten kann in der Ebene oder parallel zu der Ebene der Wundauflagefläche (313) des Wundverbandes (300) verlaufen und dass die lichtleitenden Fasern (310,610) an der Seite, die von der Wunde weg zeigt, Ausnehmungen (623) aufweisen, die sich in den Kern (521, 621) der lichtleitenden Faser (310,610) hinein erstrecken und jeweils eine in der Richtung von der Beleuchtungsvorrichtung zur Sensoranordnung (313) hin abfallende Rampe (624) aufweisen, wobei die Steilheit des Abfalls der jeweiligen abfallenden Rampe (624) in der Richtung von der Beleuchtungsvorrichtung zur Sensoranordnung (314) hin von Ausnehmung (623) zu Ausnehmung (623) monoton wächst.

6. Wundverband (100,200,300) nach einem vorstehenden Anspruch, wobei der Wundverband mindestens eine lichtleitende Referenzfaser (116,216,316) ohne Abschnitt, in dem Licht in Richtung der Wunde austreten kann, aufweist, an deren einem Ende eine Beleuchtungsvorrichtung und an deren anderem Ende eine Sensoranordnung (114,214,314) angeordnet ist.

## Claims

1. Wound dressing (100, 200, 300) with a wound contact surface (113, 213, 313), with at least one illumination device for illuminating a wound and with at least one sensor arrangement (114, 214, 314), wherein the wound contact surface (113, 213, 313) is at least partially formed by light-transmitting fibers (110, 210, 310), which are an integral component of the fabric forming the wound contact surface (113, 213, 313), as well as of its warp threads and/or weft threads, which extend through the wound contact surface (113, 213, 313) such that they completely traverse the wound contact surface (113, 213, 313) in one direction, which lies on the plane of or is parallel to the wound contact surface (113, 213, 313), and from which the light is directly and at numerous points along its course coupled out in the direction of the wound, so that light can be emitted, at least along segments, in the direction of the wound, and wherein an illumination device is arranged on one end of at least some of these light-transmitting fibers (110, 210, 310) and on its other end a sensor arrangement (114, 214, 314) is disposed and each sensor arrangement (114, 214, 314) detects the illumination intensity at the end of the light-transmitting fiber (110, 210, 310) to which it is assigned for an evaluation of the refractive index of the wound.

2. Wound dressing (100, 200, 300) in accordance with claim 1, wherein at least some of the light-transmitting fibers (110, 210, 310), from which light can be emitted, at least in segments, in the direction of the wound, can be modified such that the probability of light being emitted from the light-transmitting fibers (110, 210, 310) increases in the direction of the wound from the side on which the illumination device is arranged to the side on which the sensor arrangement (114, 214, 314) is disposed.

3. Wound dressing (100, 200) in accordance with claim 2, wherein at least the light-transmitting fibers (110, 210), on the one end of which an illumination device is disposed and on the other end of which a sensor arrangement (114, 214) is disposed and from which light can be emitted, at least in segments, in the direction of the wound, extend wavelike and vertically to the wound contact surface (113, 213) of the wound dressing (100, 200), wherein their curvature at the maxima (120, 220) and at the minima (120, 220) of the wave shape grows monotonically in the direction from the illumination device to the sensor arrangement (114, 214).

4. Wound dressing (300) in accordance with claim 2,
wherein the light-transmitting fibers (310, 710, 810) from which light can be emitted, at least in segments, in the direction of the wound, extend on the plane or parallel to the plane of the wound contact surface (313) of the wound dressing (300) and wherein the fiber material (722, 822) either comprises, in the direction of the wound, an opening (824), the extent of which grows, vertically to the running direction of the light-transmitting fibers (810), from the illumination device to the sensor arrangement (313), or comprises numerous openings (724), each surface of which monotonically grows in the direction from the illumination device to the sensor arrangement (314).

5. Wound dressing (300) in accordance with claim 2,
wherein the light-transmitting fibers (310, 610) from which light can be emitted, at least in segments, in the direction of the wound, extend on the plane or parallel to the plane of the wound contact surface (313) of the wound dressing (300) and wherein the light-transmitting fibers (310, 610) comprise, on the side facing away from the wound, recesses (623) that extend into the core (521, 621) of the light-transmitting fiber (310, 610) and of which each comprises a slope (624) descending in the direction from the illumination device to the sensor arrangement (313), wherein the steepness of descent of each descending slope (624) grows monotonically in the direction from the illumination device to the sensor arrangement (314) from recess (623) to recess (623).

6. Wound dressing (100, 200, 300) in accordance with any of the preceding claims, wherein the wound dressing comprises at least one light-transmitting reference fiber (116, 216, 316) without a segment from which light can be emitted in the direction of the wound, on one end of which an illumination device is arranged and on the other end of which a sensor arrangement (114, 214, 314) is disposed.

## Revendications

1. Pansement (100, 200, 300) comportant une surface en appui sur la plaie (113, 213, 313) ayant au moins un dispositif d'éclairage pour éclairer la plaie et au moins un dispositif de capteur (114, 214, 314), - la surface venant en appui sur la plaie (113, 213, 313) étant formée au moins en partie de fibres optiques (110, 210, 310) qui font intégralement partie du tissu formant la surface venant en appui sur la plaie (113, 213, 313) et forment ses fils de chaîne et/ou de trame, qui traversent la surface venant en appui sur la plaie (113, 213, 313) de façon à traverser la surface venant en appui sur la plaie (113, 213, 313) complètement dans une direction qui est dans le plan de la surface en appui sur la plaie ou parallèlement à cette surface et à partir desquelles de la lumière est découplée en direction de la plaie, directement et à un ensemble de positions le long de leur tracé, de façon qu'au moins par segments, la lumière puisse être émise en direction de la plaie, pour qu'au moins certaines de ces fibres optiques (110, 210, 310) comportent à l'une de leurs extrémités, un dispositif d'éclairage et à l'autre extrémité, un dispositif de capteur (114, 214, 314) et en ce que pour l'exploitation de l'indice de calcul de la plaie, le dispositif de capteur (114, 214, 314) respectif détecte l'intensité du rayonnement à l'extrémité des fibres optiques (110, 210, 310) à laquelle il est associé.

2. Pansement (100, 200, 300) selon la revendication 1,
dans lequel
au moins certaines des fibres optiques (110, 210, 310) à partir desquelles la lumière peut sortir au moins par segments en direction de la plaie, sont modifiées de façon à augmenter la probabilité que la lumière sorte des fibres optiques (110, 210, 310) en direction de la plaie par le côté muni du dispositif d'éclairage vers le côté muni du dispositif de capteur (114, 214, 314).

3. Pansement (100, 200) selon la revendication 2,
dans lequel
au moins les fibres optiques (110, 210) dont une extrémité comporte un dispositif d'éclairage et l'autre extrémité, un dispositif de capteur (114, 214) et à partir desquelles la lumière sort au moins par segments en direction de la plaie, ont un tracé ondulé dans la direction perpendiculaire à la surface en appui sur la plaie (113, 213) du pansement (100, 200),
la courbure au niveau des maximums (120, 220) et des minimums (120, 220) a une croissance monotone pour la forme ondulée en direction du dispositif d'éclairage vers le dispositif de capteur (114, 214).

4. Pansement (300) selon la revendication 2,
dans lequel
les fibres optiques (310, 710, 810) dont la lumière peut sortir au moins par segments en direction de la plaie, sont dans le plan ou parallèlement au plan de la surface en appui sur la plaie (313) du pansement (300), et
l'enveloppe de fibres (722, 822) présente une ouverture (824) en direction de la plaie et dont l'extension dans la direction perpendiculaire à la direction de tracé des fibres optiques (810) à partir du dispositif d'éclairage vers le dispositif de capteur (313) augmente ou comporte plusieurs ouvertures (724) dont les surfaces respectives augmentent de façon monotone dans la direction allant du dispositif d'éclairage vers le dispositif de capteur (314).

5. Pansement (300) selon la revendication 2,
**caractérisé en ce que**
les fibres optiques (310, 610) dont la lumière peut sortir au moins par segments en direction de la plaie, sont dans le plan ou parallèlement au plan de la surface en appui sur la plaie (313) du pansement (300), et
les fibres optiques (310, 610) sur le côté qui est non tourné vers la plaie, ont des évidements (623) qui arrivent dans le cœur (521, 621) des fibres optiques (310, 610) et ont une rampe (624) descendant dans la direction allant du dispositif d'éclairage vers le dispositif de capteur (314), la pente de la rampe descendante respective (624) dans la direction allant du dispositif d'éclairage vers le dispositif de capteur (314) ayant une croissance monotone à partir d'un évidement (623) vers un évidement (623).

6. Pansement (100, 200, 300) selon l'une des revendications précédentes,
ayant au moins une fibre optique de référence (116, 216, 316) sans segment par lequel la lumière peut sortir en direction de la plaie et dont une extrémité a un dispositif d'éclairage et l'autre extrémité a un dispositif de capteur (114, 214, 314).
